# EUROPÄISCHE PATENTSCHRIFT

(11) **EP 1 916 973 B1**
(45) Veröffentlichungstag und Bekanntmachung des Hinweises auf die Patenterteilung: **06.10.2010**
(21) Anmeldenummer: 06829393.5
(22) Anmeldetag: 07.12.2006
(51) Int. Cl.: A61F 7/00, A61H 33/00

(54) **VORRICHTUNG ZUR DURCHFÜHRUNG EINER ANWENDUNG**
DEVICE FOR CARRYING OUT A TREATMENT
DISPOSITIF EN VUE DE L'EXECUTION D'UNE APPLICATION

(30) Priorität: 09.12.2005 DE 102005059389
(43) Veröffentlichungstag der Anmeldung: 07.05.2008
(73) Patentinhaber: Haslauer, Paul, A-5020 Salzburg (AT)
(72) Erfinder: Haslauer, Paul, A-5020 Salzburg (AT)
(74) Vertreter: Flach, Dieter Rolf Paul
(86) Internationale Anmeldenummer: PCT/EP2006/011777
(87) Internationale Veröffentlichungsnummer: WO 2007/065687

(56) Entgegenhaltungen:
- EP-A- 1 529 512
- EP-A1- 0 144 571
- DE-A1- 2 444 611
- DE-U1- 8 816 075

## Beschreibung

Die Erfindung betrifft eine Vorrichtung zur Durchführung einer Anwendung nach dem Oberbegriff des Anspruches 1.

Wärmebehandlungen, beispielsweise in Form von Packungen, Badeanwendungen oder auch in Form von Dampfbad- oder Kräuterdampfbadanwendungen, erfreuen sich seit jeher großer Beliebtheit. Derartige Anwendungen dienen nicht nur der Verbesserung des allgemeinen Wohlbefindens, sondern können auch zur gesundheitlichen Stabilisierung und Verbesserung beitragen.

Bei derartigen Wärmeanwendungen sind z.B. zwei Aspekte zu berücksichtigen.

Zum einen ist bekannt, dass Badeanwendungen, insbesondere in Kurbädern, Wellness-Einrichtungen, Hotels und dergleichen durchaus nicht zu vernachlässigende Kosten verursachen. Denn die verwendeten Bademedien müssen schon aus Hygienegründen nach Durchführung einer Anwendung entfernt und für die Durchführung einer nächsten Anwendung durch hygienisch einwandfreie Bademedien ersetzt werden. Sollen zudem besonders wirksame Naturstoffe oder sonstige Zusätze verwendet werden, so sind auch hier in der Regel beachtliche Mengen erforderlich, die die Kosten einer derartigen Anwendung nochmals drastisch erhöhen.

Zum anderen ist aber auch zu berücksichtigen, dass bedingt durch die deutlich erhöhte Lebenserwartung weiter Bevölkerungskreise bei der Durchführung von Wärmeanwendungen, insbesondere bei älteren Menschen, eine entsprechende Schonung des Herz-Kreislauf-Systems nicht unberücksichtigt bleiben darf. Großflächige und dabei zu stark überwärmende Anwendungen können problematisch sein.

Um die benötigte Bademenge, beispielsweise die Menge an Peloid oder an Badezusätzen zu verringern, ist gemäß der DE-PS 30 46 628 bereits eine verbesserte Vorrichtung zum Verabreichen von Peloid-Voll- bzw. -Sitzbädern vorgeschlagen worden, bei der für jede einzelne Badetherapie nur ein geringerer Teil der bisher des aus Hygienegründen nur einmal verwendungsfähigen Bademediums erforderlich ist. Dies wurde dadurch gelöst, dass ein Einsatz in der Wanne vorgesehen ist, der als flüssigkeitsdichte Folie zur Trennung der oberhalb und unterhalb der Folie einzubringenden Peloid-Badeflüssigkeit ausgebildet ist. Bei Durchführung einer Badeanwendung kann sich somit jemand in den Raum oberhalb der flüssigkeitstrennenden Folie hineinlegen, so dass nur noch die mit ihm in Kontakt tretenden Badeflüssigkeit aus Hygienegründen ausgewechselt werden muss. Dadurch lässt sich gegenüber herkömmlichen Anwendungen das Volumen an Badeflüssigkeit und Badezusätzen, Peloid etc. deutlich verringern. Das Problem einer möglichen Überwärmung des Körpers bleibt aber bestehen.

Aus der EP 0 144 571 B1 ist ferner eine Vorrichtung zum Verabreichen von Packungen bekannt geworden, bei der die Packungen nur an bestimmten zu überwärmenden Positionen bei Durchführung einer Anwendung positioniert werden, wobei sich durch Verwendung von Isoliermatten vor allem im Bein- und Nackenbereich der Vorteil ergibt, dass hier vor allem auch bei älteren Menschen, eine Überwärmung von einem unterhalb einer Auflage- und Anpressfolie dienenden Wärme- und Anpressmedium vermieden werden kann.

In beiden vorstehend genannten vorbekannten Wannenanordnungen wird also jeweils eine Folie verwendet, die am Wannenumlaufrand fest eingespannt und gehalten ist. Bei der Folienwanne gemäß der EP 0 144 571 B1 wird bei der Verabreichung von Packungen ferner vorgeschlagen, aus Hygienegründen eine Einmalfolie zusätzlich zu verwenden. So können bei der aus der vorstehend genannten Vorveröffentlichung bekannten Wanne auf die Andrückfolie zunächst an den Stellen, an denen eine Überwärmung vermieden werden soll, Isoliermaterialien aufgelegt werden (beispielsweise auch im Hals-, Nacken- oder Kopfbereich in Form von Tüchern oder Handtüchern), um dann darauf letztlich die Einmalfolie aufzulegen, die also abschnittsweise auf der Andrückfolie zu liegen kommt und abschnittsweise auf den zuvor aufgelegten Isoliermaterialien. Darauf legt sich dann eine zu behandelnde Person auf, an deren Körperoberfläche die jeweils gewünschten Packungen oder breiigen oder schmierfähigen Medien aufgetragen werden können. Nach der Behandlung wird die Einmalfolie weggeworfen.

Eine Folienwanne sowie ein Verfahren zur Bereitstellung einer derartigen Wanne ist auch aus der EP 1 529 512 A2 bekannt geworden. Es handelt sich hierbei um eine sogenannte Körperformwanne, bei der der Wannenquerschnitt an die rückwärtige Körperform eines Badenden angepasst ist. Zur Durchführung des Badens wird eine lose Folie eingelegt, auf die sich der Badegast legen kann. Die betreffende Person liegt also auf der Folie unmittelbar auf, die aufgrund des Körpergewichts bei entsprechender Auflage des Körpers am Wannenboden und teilweise an dem seitlichen Übergangsbereich zum Wannenseitenbereich anliegt. Als nächstes kann dann beispielsweise eine Badeflüssigkeit eingefüllt werden, und zwar auf der Folienoberseite. Als nächster Schritt kann die Wanne in der Regel mit heißem Wasser befüllt werden, und zwar in dem Bereich unterhalb der Folie, bis durch die Befüllung der Wanne die Auftriebskräfte auf den Körper wirken. Die Folie legt sich dabei durch den Wasserdruck am Körper an. Schließlich kann die Folie durch eine weitere Person über den Folienrand leicht hinausgezogen werden, wodurch der Badende in dem befüllten Wasser leicht angehoben wird. Die Folie liegt dabei aber immer eng am Körper an. Durch die schwimmende Situation führt die Folie entsprechende Körperbewegungen mit.

Eine Vorrichtung unter Verwendung einer Folie ist beispielsweise aus dem DE 88 09 687 U1 bekannt geworden. Es handelt sich dabei um einen Kunststoffeinsatz für eine Sitzbadewanne in Form einer Folie, die passgenau in die Sitzbadewanne eingelegt wird und nach deren Gebrauch leicht zu entfernen ist, ohne dass größere Zeitverluste durch aufwendige Reinigungs- oder Desinfektionsarbeiten anfallen sollen.

Demgegenüber ist es Aufgabe der vorliegenden Erfindung, eine neue Vorrichtung zu schaffen, die neue Möglichkeiten der Verabreichung einer Anwendung eröffnet.

Die Aufgabe wird erfindungsgemäß entsprechend den im Anspruch 1 angegebenen Merkmalen gelöst. Vorteilhafte Ausgestaltungen der Erfindung sind in den Unteransprüchen angegeben.

Durch die vorliegende Erfindung wird eine völlig neue Vorrichtung konzipiert, mit der eine völlig neuartige Anwendung durchgeführt werden kann.

Die erfindungsgemäße Vorrichtung zeichnet sich nämlich dadurch aus, dass sie eine bevorzugt aus einer Folie bestehende Auflagefläche aufweist, die auch bei Durchführung der Anwendung, also bei einer sich auf der Folie befindlichen Person, nur vergleichsweise wenig nach unten durchhängt. Dadurch wird eine eher schalen-, teller- oder wokähnliche Auflagefläche gestaltet, auch wenn die Folie an einer wannenähnlichen Tragvorrichtung, nämlich an einem umlaufenden Rand der Wanne zumindest abschnittsweise gehalten ist.

Mit anderen Worten soll die Auflagefläche oder Folie auch im belasteten Zustand während der Durchführung der Anwendung beispielsweise seitlich vom Körper aus weg verlaufend nur in einem vergleichsweise kleinen Winkel gegenüber der Horizontalen ansteigen, der nicht größer als 45° und vorzugsweise kleiner als 40°, 35°, 30° oder sogar kleiner als 25° sein kann. Dadurch ergibt sich eine vergleichsweise nur leicht schalig oder konkav durchgebogene Auflagefläche, deren außenliegender Halterand, an welchem die Folie getragen und abgestützt ist, in einer Höhe liegt, auf der auch die auf der Folie befindliche Person liegt. Mit anderen Worten kann der Rand der Haltefolie in einem geringen Abstand oberhalb der auf der Folie befindlichen Person oder auch in Höhe der auf der Folie liegenden Person, beispielsweise in Höhe des Kopf-, Brust- oder Bauchbereiches liegen. Auch wenn die Folie am Außenrand an anderer Stelle abgestützt ist, ist zumindest eine Umlenkstelle vorgesehen, die relativ zu der auf der Folie liegenden Person in einer Höhe so angeordnet ist, dass die Folie zu dieser Umlenkstelle vom Körper aus, also von der Stelle, wo die Folie am stärksten aufgrund der Belastung durchhängt, nur vergleichsweise langsam und wenig ansteigt, um nur eine vergleichsweise gering gestaltete vertiefte Auflagefläche zu gewährleisten, in der die betreffende Person leicht gleiten und rutschen kann.

Durch die Erfindung lässt sich eine wohltuende, gewissermaßen streichelnde und/oder massierende Anwendung durchführen. Eine derartige Behandlung war bisher nur passiv dadurch möglich, dass eine zweite Person, beispielsweise ein Masseur oder ein Therapeut, am Körper eines Gastes oder Patienten eine Massage durchgeführt hat.

Im Gegensatz dazu schafft die Vorrichtung eine Möglichkeit, in der der Badegast selbst durch von ihm selbst initiierte Körperbewegungen durch die dadurch initiierte Gleit- und Reibwirkung die Anwendung selbst durchführen und steuern kann.

Aber auch wenn ein Teil der Badegäste die Anwendung nicht nur eigenverantwortlich und quasi selbststeuernd durchführen will, sondern auch hier eine gewisse Passivität bevorzugt, bietet die Erfindung ergänzende Möglichkeiten. So kann beispielsweise die den Gast tragende Folie, die nachfolgend auch teilweise Gleitfolie genannt wird, seitlich oder fußseitig weggezogen und wieder in die gleiche Position gebracht werden. Dies kann beispielsweise durch eine oder mehrere verfahrbare Abstütz- oder Aufhängeeinrichtungen, beispielsweise in Form von seitlichen Rollen gewährleistet werden, worüber die Folie z.B. von links nach rechts und umgekehrt hin und her bewegt werden kann. Alternativ oder ergänzend ist es auch möglich, unterhalb der Folie zusätzliche einflussnehmende Einrichtungen vorzusehen, um die Folie in ihrer Lage und damit den darauf befindlichen Gast zu bewegen. Auch eine Veränderung des Schwerpunkts der Trag- oder Wanneneinrichtung beispielsweise durch Anheben, Absenken, Kippen um eine in Längsrichtung der Trag- und Wanneneinrichtung oder um eine in Querrichtung verlaufende Kipp- und/oder Verschwenkachse und/oder Drehen um eine eher vertikale Achse ist möglich.

Eine derartige Ausgestaltung eröffnet nunmehr die Möglichkeit, unter Verwendung von beigebbaren Medien, die fließfähig sind, pastös, breiig oder cremeförmig, dass eine auf dieser Folie befindliche Person auf dieser Folie bereits bei einer geringsten Körperbewegung gleitet und rutscht. Die Folie führt dadurch am Körperrücken, Schulter- und Gesäßbereich reibende und gleitende Bewegungen durch. Mit anderen Worten wird im Rahmen der Erfindung eine Vorrichtung in Form eines Auflagers, Liege etc. geschaffen, mit dem Ziel der Förderung und/oder der Ermöglichung eines gleitenden Kontakts unter Verwendung eines Gleitmediums.

Auch unterhalb der Folie vorgesehene an- und/oder abhebbare, auf die Folienunterseite einwirkende Trag- und/oder Abstützeinrichtungen, beispielsweise auch in Form von gepolsterten und/oder aufblasbaren Einrichtungen sind denkbar. In einem geschlossenen Folienraum kann beispielsweise auch fließfähiges oder gasförmiges Medium wie Luft eingeblasen oder eingepumpt werden, wodurch unterschiedliche Druckbelastungen ausübende Einflüsse erzeugt werden können, die die gewünschten Effekte bei Durchführung der Anwendung erzeugen und/oder verstärken.

Im Rahmen der Erfindung kommt dem sog. "Wok-Effekt" eine Bedeutung zu. Aufgrund der spezifischen schalen- oder tellerförmigen Wok-Querschnittsgestaltung führt dies bekanntermaßen dazu, dass beispielsweise auf die Folie gegebene Anwendungsstoffe und Substanzen, die fließfähig, cremeförmig, pastös etc. sein können, der Schwerkraft folgend vor allem zum tiefsten Punkt in Mitte der Folie fließen oder gleiten. Durch das Einsteigen des Gastes wird die Gleitfolie nach unten gedrückt. Es entsteht an allen Seiten eine Schräglage der Folie. Durch die gleitenden Bewegungen des Gastes und durch sein Körpergewicht wird ein Großteil des zuvor auf dem Körper des Gastes und/oder auf der Folienoberfläche aufgetragenen Gleitmediums seitlich zwischen Körper und Folie herausgedrückt. Dieser Teil bewegt sich durch den Wok-Effekt zwangsweise immer wieder in Kontakt zwischen Körper und Folie. Der Gast kann hier quasi "überschüssiges" Gleitmaterial von Hand nehmen und zur Einsalbung seinem Körper gezielt zuführen. Dadurch wird die Menge an aufgebrachten Einmal-Material weiter reduziert.

Um die Funktionsfähigkeit der Folie und deren langlebige Benutzung zu verbessern, kann die Folie auch als Doppel- oder Mehrlagenfolie ausgebildet sein.

Allgemein gesprochen sollte die Folie zur Aufnahme der Körpergewichte eine ausreichend starke und tragfähige Schicht umfassen, beispielsweise mit einer Gewebeverstärkung versehen sein, so dass die Folie auf jeden Fall mit ausreichender Sicherheit die Tragkräfte einer Person aufnehmen kann. Eine so gebildete Folie kann an ihrer Folienoberseite mit einer möglichst gut gleitenden Schicht versehen, kaschiert oder allgemein ausgestattet sein. Zwischen der obersten gleitenden Schicht und der darunter befindlichen Tragschicht kann auch ein Hohlraum gebildet sein, der noch mit einem fließfähigen, gasförmigen oder gelähnlichen Material befüllt ist, wodurch auch eine Niveauausgleichung der obersten Gleitschicht bewerkstelligbar ist. Selbst bei Durchhängen der Tragschicht kann dadurch gewährleistet werden, dass die obere Gleitfolie quasi horizontal zu liegen kommt.

Es kann aber auch noch eine weitere eher durchhängende Folie unterhalb der Tragfolie vorgesehen sein, wodurch vorzugsweise ebenfalls wieder ein geschlossenes Kammersystem gebildet wird. Führt in diese Kammer zumindest eine Zuführöffnung hinein und gegebenenfalls noch eine andere ergänzende, aber nicht notwendige Ablauföffnung heraus, so kann der untere Hohlraum beispielsweise mit heißer Luft als Wärmemedium durchströmend befüllt werden. Auch diese unterste Folie wird bevorzugt gewebearmiert, um als Sicherheit zu dienen, wenn die obere Tragfolie aus unerwarteten Gründen reißen sollte. Auf jeden Fall wird durch dieses untere Kammersystem eine geschlossene Beheizungskammer gebildet, so dass auf einen Wannenkörper insgesamt verzichtet werden kann. Lediglich eine Tragvorrichtung für die Folienkonstruktion ist voll ausreichend, um auch die gewünschten Temperaturen bei Durchführung der Anwendung einstellen zu können, während des Verlaufs der Anwendung aufrecht zu erhalten oder auch während des Verlaufs der Anwendung zu verändern. Auch wenn also eine auf der obersten Gleitfolie aufliegende Person diese Folie bis zur Berührung der darunter befindlichen, vorzugsweise gewebearmierten Tragfolie durchdrücken sollte, bleibt der gewünschte Wärmetransport und die Erwärmung insgesamt aufrecht erhalten, weil die unterste Kammer nicht zusammengepresst werden kann. Um einen guten Befüllungsdruck des Wärmemediums in der untersten Kammer aufrecht zu erhalten, kann im Ablauf eine vorzugsweise einstell- oder verstellbare Drossel oder eine Austrittsöffnung mit geringerem Durchmesser als die Eintrittsöffnung vorgesehen sein.

Schließlich bietet dieses System auch den Vorteil, dass vor der Anwendung durch Einpressen von warmer Luft die oberste und die zweite Folie konvex etwas nach oben aufgebaucht werden, wodurch die Reinigung stark vereinfacht wird (weil z.B. Reinigungsflüssigkeit). Legt sich ein Badegast auf die oberste Folie, so wird die erste und zweite Folie entsprechend nach unten gedrückt, jedoch nicht soweit, dass die dritte und damit unterste Folie berührt wird. Dieses Mehrfach-Kammersystem dient also der Erfüllung einer Doppelfunktion, nämlich zum sicheren Tragen und Halten eines Badegastes auf der obersten Folie und zur Bereitstellung eines beheizbaren Raumes darunter.

Eine unerwünschte Überwärmung des Kopfes kann ferner durch eine spezifische Kopfauflage einschließlich einer Kopf- und Nackenstücke realisiert werden.

Zudem können auch auf- und/oder umklappbare Griffe oder zumindest in Reichweite positionierbare Griffe oder sonstige Halteeinrichtungen bevorzugt seitlich an der Vorrichtung vorgesehen sein, die es dem Badenden oder Gast ermöglichen, sich hieran mit seinen Händen abzustützen und möglicherweise auch durch Muskelbetätigung der Arme seinen Körper entsprechend in der leichten Vertiefung der Folie hin und her gleiten zu lassen, zu schieben, zu drehen oder in sonstiger Weise zu drehen. Gleichzeitig wird dadurch auch ein unerwünschtes Herausgleiten aus der Folienauflage unterbunden und ein gezieltes Ein- und Aussteigen erleichtert. Soll ein zu tiefes Hineinrutschen in die schalen- oder wokförmige Auflagefläche verhindert werden, so kann beispielsweise auch ein verstellbares Fußraster vorgesehen sein, was insbesondere für kleinere Gäste den Komfort erhöht.

Schließlich können auch auf der Folie noch einige Keile, seitlich Stützungskeile etc. positioniert werden, die die Gleitbewegung zumindest in gewissem Maße begrenzen und einschränken, um gegebenenfalls auch ein Angstgefühl bei dem einen oder anderen Badegast zu verhindern. Diese gegebenenfalls, den Körper zumindest leicht stützende Zusatzeinrichtung in Form von keilförmigen Polstern, aufblasbaren Körpern etc. können beispielsweise durch geeignete Vorrichtungen mit der die Folie tragenden Vorrichtung insgesamt verbunden sein, so dass sie ein- und ausgeschwenkt werden können, ohne verloren zu gehen. An diesen zusätzlich positionierbaren Keilen oder Begrenzungskörpern können auch noch zusätzliche Handgriffe oder Handgreifabschnitte ausgebildet sein, an dem sich ein Badegast festhalten kann. Schließlich kann auch die Körperstellung und -haltung durch Verwendung derartiger Keile verändert werden, beispielsweise durch ein keilförmiges Auflagepolster, welches unter leicht angehobene und abgewinkelte Kniee gelegt werden kann, oder beispielsweise durch ein Auflagepolster im oberen Schulter- oder angehenden Rückenbereich etc.

Weitere Vorteile, Einzelheiten und Merkmale der Erfindung ergeben sich nachfolgend aus dem anhand von Zeichnungen dargestellten Ausführungsbeispiel. Dabei zeigen im Einzelnen:
- Figur 1 :: eine schematische dreidimensionale Dar- stellung einer Vorrichtung mit straff ge- zogener Auflage- bzw. Gleitfolie auf der Oberseite, die abweichend zur erfindungs- gemäßen Ausgestaltung der Auflage- und Gleitfolie nicht schalen-, teller- oder wokähnlich, sondern lediglich nur bezogen auf die Querrichtung der Vorrichtung kon- kav gestaltet ist;
- Figur 2 :: eine schematische, in Vertikalrichtung verlaufende Querschnittsdarstellung quer zur Längsrichtung der Vorrichtung;
- Figur 3 :: eine entsprechende Darstellung zu Figur 2, jedoch bei einer auf der Auflage- bzw. Gleitfolie aufgelegten Person;
- Figur 4 :: eine schematische Draufsicht während der Durchführung einer Anwendung unter Verwen- dung von zusätzlichen Hilfseinrichtungen;
- Figur 5 :: eine schematische Seitenansicht der Vor- richtung unter Darstellung eines Innen-
- Figur 6 :: und/oder Knieteils mit daran sitzenden Greifeinrichtungen; eine weitere seitliche Darstellung unter Andeutung eines Knieteils und eines Rüc- kenstückteils zur Veränderung der Liegepo- sition eines Badegastes; und
- Figur 7 :: eine Querschnittsdarstellung durch eine mehrwandige Gleitfolie unter Ausbildung von Folienzwischenräumen.

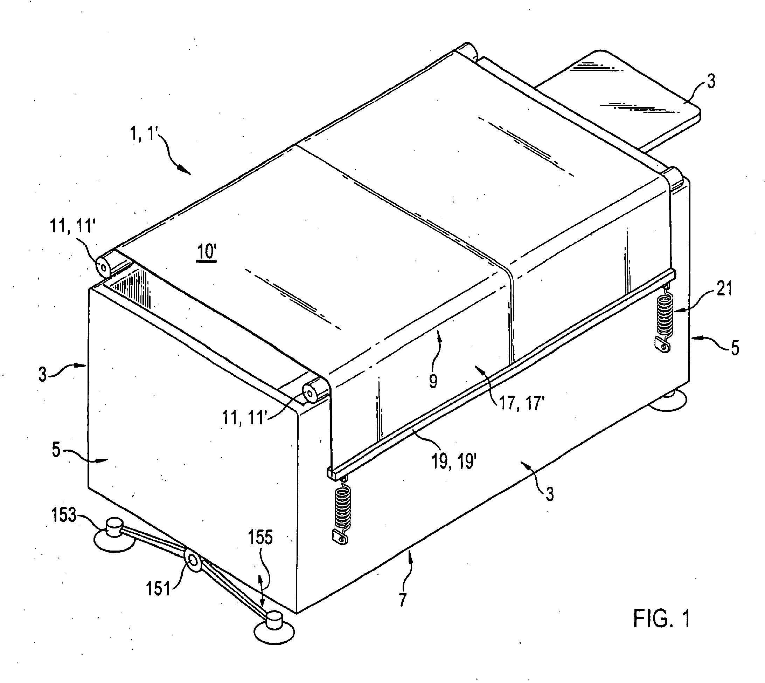

In den Figuren 1 bis 3 ist ein Beispiel für den Grundaufbau einer erfindungsgemäßen Vorrichtung gezeigt, die allerdings bei dem gezeigten Beispiel im Gegensatz zu der erfindungsgemäß schalen-, teller- oder wokähnlich gestalteten Folie nur ein grundsätzliches Beispiel zeigt, bei der die Folie nur in Querrichtung der Vorrichtung konkav und nicht auch in Längsrichtung der Folie konkav gestaltet ist, wodurch sich erst die schalen-, teller- oder wokähnliche Gestaltung der Auflagefläche der Folie ergibt.

Es ist eine Tragvorrichtung 1 vorgesehen, die im gezeigten Ausführungsbeispiel aus einem Wannengrundkörper 1' mit zwei gegenüberliegenden Längsseiten 3 und zwei ebenfalls in Längsrichtung beabstandeten Stirnseiten 5 besteht. Die Tragvorrichtung 1 hat einen Boden 7 und ist im oberen Randbereich offen.

Im gezeigten Ausführungsbeispiel sind in Höhe oder zumindest näherungsweise im Bereich des oberen umlaufenden Randes 9 an der Tragvorrichtung in Form des Wannengrundkörpers 1', d.h. im gezeigten Ausführungsbeispiel an den beiden Längsseiten 3, Umlenkeinrichtungen 11 vorgesehen, die im gezeigten Ausführungsbeispiel aus Umlenkrollen 11' bestehen. Die Umlenkeinrichtung 11 kann auch aus dem oberen Rand 9 der Trageinrichtung 1 bestehen. Drehbare Rollen sind jedoch bevorzugt, da hierdurch die Folie 10 (worauf nachfolgend noch genauer eingegangen wird) leichter von links nach rechts und umgekehrt hin und her verfahren werden kann. Die Achslagerung der Rollen 11 ist im gezeigten Ausführungsbeispiel nicht näher gezeigt. Die Abstützung dieser Umlenkeinrichtung vorzugsweise in Form der Umlenkrollen 11' kann im Bereich des oberen Randes 9, an der Innen- oder Außenseite 3 des Wannenkörpers oder auch außerhalb des Wannenkörpers an einer Stand- oder Bodenfläche 15 erfolgen und dort abgestützt sein.

Die Umlenkrollen 11' können dabei bevorzugt weich oder elastisch gestaltet sein, um insbesondere Verletzungsgefahren beim Ein- und Aussteigen zu vermeiden.

Im gezeigten Ausführungsbeispiel ist ersichtlich, dass die erwähnte Auflage- und/oder Gleitfolie 10, die am Wannenrand über die Umlenkeinrichtung 11 aus dem Bereich der Wanne herausgeführt und außen an der Wanne nach unten weitergeführt ist, im Bereich der Wanne nicht über eine relative Höhe durchhängt oder gar bis in die Nähe des Wannenbodens reichen würde. Vielmehr ist das Ausführungsbeispiel so gewählt, dass die Gleitfolie 10 zumindest in ihrer Ausgangssituation über einen Verankerungsabschnitt 17 vorzugsweise an einem außen liegenden Randbereich 17' über eine Halte- oder Verankerungseinrichtung 19 vorzugsweise in Form eines schienenförmigen Spannrahmens 19' über eine Betätigungs- und/oder Abstützeinrichtung 21 gehalten ist.

In dem Ausführungsbeispiel gemäß Figur 1 ist dabei angedeutet, dass die Abstützeinrichtung 21 eine Spannvorrichtung 21' umfasst, die beispielsweise eine Federeinrichtung 21' oder eine Federkraftspeichereinrichtung 21" beispielsweise in Form von einem oder mehreren Schraubenfedern umfassen kann, die an geeigneter Stelle gehalten oder abgestützt ist. Dadurch wird in der Ausgangslage sichergestellt, dass die erwähnte Auflagen- und/oder Gleitfolie 10 in unbelastetem Zustand oberhalb des Wanneninnenraumes 1" quasi völlig gestreckt und damit quasi nicht durchhängend verläuft.

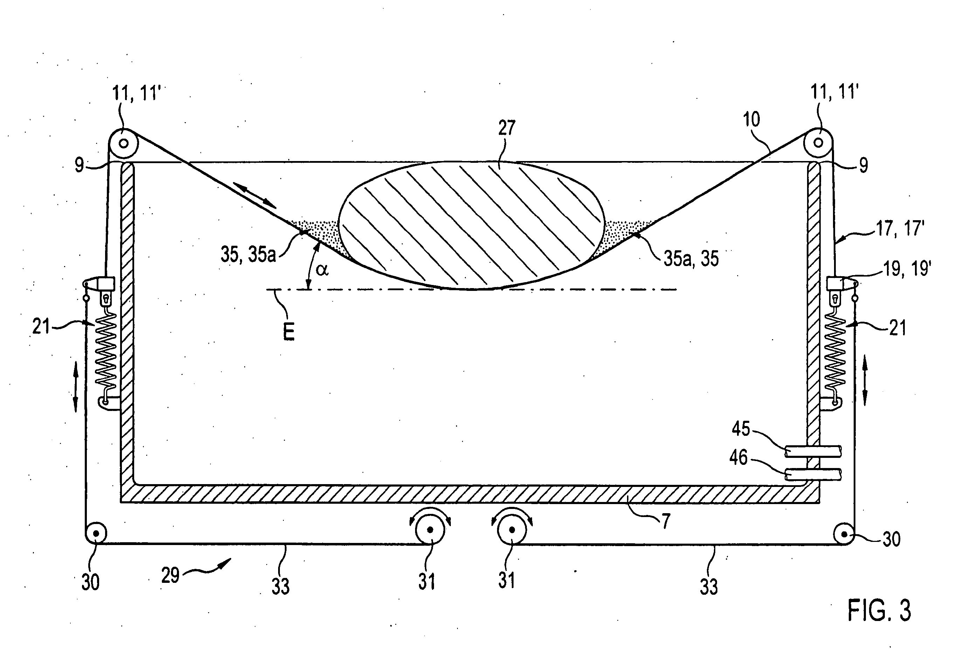

Die Federkraftspeichereinrichtung 21" ist dabei so dimensioniert, dass bei Aufliegen einer Person dann die Auflagen- und Gleitfolie 10 vergleichbar der Querschnittsdarstellung nach Figur 3 quasi nur geringfügig in den Innenraum 1" der Tragvorrichtung vorzugsweise in Form des Wannengrundkörpers 1' eintaucht.

Im gezeigten Ausführungsbeispiel ist dabei die Federkraftspeichereinrichtung 21" bevorzugt so abgestimmt, dass für ein durchschnittliches Körpergewicht die Folie seitlich von dem auf der Folie befindlichen Gast oder der die Anwendung durchführenden Person 27 nur vergleichsweise flach zu der Abstütz- und Umlenkeinrichtung 11 ansteigt, vorzugsweise in einem Winkel α gegenübereiner Horizontalebene E, der kleiner ist als 45°, vorzugsweise kleiner als 40°, 35° und insbesondere kleiner als vorzugsweise 25°, 20°, 15°, 10°, 5°. Dadurch wird also eine nur ganz leicht konkav ausgeformte Auflagefläche 10' gebildet.

Nachfolgend wird auf die Durchführung der Anwendung im Zusammenhang mit weiteren Ausgestaltungen der Vorrichtung eingegangen.

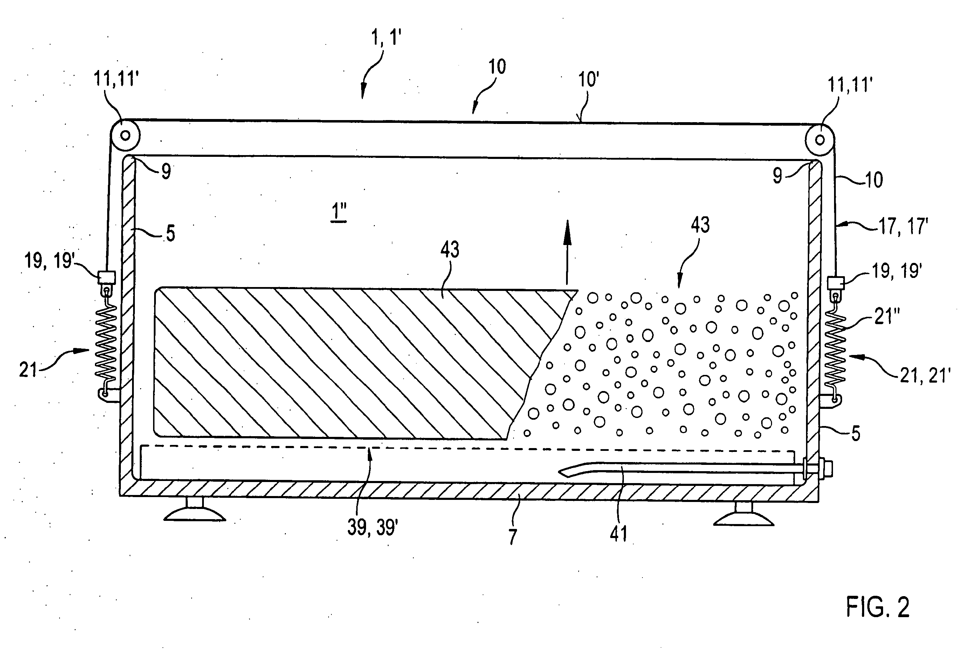

In der in Figur 1 und 2 gezeigten Ausgangs-Bereitsschaftsstellung ist also die obenliegende Gleitfolie 10 mehr oder weniger flächig und eben, d.h. in ihre zumindest näherungsweise horizontale Ausgangsstellung gespannt. In dieser Stellung ist es nunmehr beispielsweise für einen Bademeister, Therapeut, Masseur oder sonstige Hilfsperson leicht, entsprechende Medien auf der Folie aufzubringen, allgemein also fließfähige, breiige oder pastöse, cremige oder sonstige Medien, die nachfolgend kurz auch als Gleitmedien bezeichnet werden. Im Übrigen ist es auch nach Durchführung der Anwendung in dieser wiederum erreichbaren Ausgangsstellung für die betreffende Person beispielsweise in Form des Bademeisters ein leichtes, die Oberfläche der Folie zu reinigen, da keine Rückstände oder Pfützen zurückbleiben.

Vor Durchführung der Anwendung empfiehlt sich zunächst eine sog. Initial-Salbung an einer die Anwendung durchzuführenden Person vorzunehmen, die diese selbst durchführen kann oder beispielsweise vom Bademeister durchführen lassen kann. Anschließend wird die Gleitfolie 10 beispielsweise mit einem bevorzugt warmen Medium wie Warmwasser leicht benetzt und gegebenenfalls eine dünne Schicht des Gleitmediums auf die Folie aufgetragen, aufgespritzt, verteilt, verrieben etc.

Dann kann sich der Badegast beispielsweise in Bauchlage auf die Folie legen. Da der seitliche Rand der Vorrichtung gleitfähig und die erwähnten Umlenkrollen bevorzugt gepolstert sind, ist es für den Badegast ein leichtes, bequem in die Vorrichtung einzusteigen, d.h. sich auf der Folienoberseite hinzulegen.

Wenn sich also ein Gast dann auf die Folie 10 legt, wird der Körper aufgrund eines Eigengewichtes dazu beitragen, dass sich die Folie 10 entgegen der Kraft der Federspanneinrichtung 21' zumindest geringfügig konkav durchbaucht und dadurch etwas in das Wanneninnere 1" eintaucht. Der Grad der Eintauchtiefe der Folie 10 in das Innere der in Form eines Wannengrundkörpers 1' ausgebildeten Tragvorrichtung 1 bestimmt die am Gleiten beteiligte Hautoberfläche. Der Reibungswiderstand zwischen dem Körper 27 einerseits, dem dazwischen befindlichen Gleitmedium und der Gleitfolie andererseits wird ebenfalls von der Eintauchtiefe und damit der Reibungsfläche bestimmt.

Nachdem sich also der Badegast 27 beispielsweise in Bauchlage auf der Folie platziert hat und der Körper geringfügig in das Wanneninnere 1" eintaucht, kann dann durch den mitwirkenden Bediensteten beispielsweise in Form des Bademeisters der Rücken des Badegastes mit Warmwasser befeuchtet und mit einem Teil des Gleitmediums eingeschmiert und mit großflächigen Verteilbewegungen am Körper verteilt werden.

Nach der vorstehend erwähnten Eingangs-Einsalbung des Badegastes braucht nur noch restliches Gleitmedium auf dem Rücken, Schulterpartie und Beinbereich des Badegastes aufgetragen bzw. aufgegossen zu werden. Der Badegast dreht sich nunmehr auf den Rücken. Dabei wird je nach Konsistenz des Gleitmediums 35 ein großer Teil dieses Gleitmediums aus dem Raum zwischen dem Körper 27 und der Gleitfolie 10 nach außen verdrängt, wie dies mit Bezugszeichen 35a in Figur 3 angedeutet ist. Bewegt der Badegast seinen Körper, wird restliches Gleitmedium durch die Schräglage der Gleitfolie 10 immer wieder an den Körper des Badegastes herangeführt. Für den Badegast bedeutet diese Verdrängung des überschüssigen Gleitmittels ein besonderes Erlebnis. Hier wird ein angenehmes Ansprechen der Berührungsrezeptoren auch ohne eigentliche aktive Bewegung bewirkt.

Dieses Erlebnis ist durchaus mit einer passiven Immersion zu vergleichen. Auch ohne extra darauf hinzuweisen, streicht der Badegast mit seinen Händen das Medium am Körper auf und massiert es ein. Nun kann beispielsweise eine trockene Mischung aus Kristallzucker, Heilerden und ähnliches mehr zusätzlich aufgebracht werden. Bis die Zuckerkristalle aufgelöst sind, ist ein angenehmes Peeling auf der Haut möglich. Anschließend vermengen sich die vorstehend erwähnten zusätzlich zugegebenen Stoffe wie Kristallzucker, Heilerde etc. mit dem ursprünglich aufgebrachten Gleitmedium. Dieses zeitlich begrenzte Peeling wird als sehr angenehm empfunden und pflegt die Haut. Dies führt auch dazu, dass im gesamten Gleitmedium z.B. die Salzkonzentration ansteigt. Dieses Ansteigen der Salzkonzentration ist also sog. Retard-Effekt wünschenswert. Die Beigabe von Rotuli gleich anfangs oder zu einem späteren Zeitpunkt kann einen sog. Kugellagereffekt bewirken. Dies bewirkt eine deutliche Erhöhung des Gleiteffekts. Dem Macadamia-Nussöl werden solche Eigenschaften zugesprochen. Andererseits können beigegebene Granuli (unlösliche oder lösliche Körnchen) den Widerstand erhöhen und den Peeling-Effekt ermöglichen bzw. verstärken.

Für die Anwendung wird ein entsprechendes umgebendes Klima bevorzugt. Ein Gleitbad in einem Raum mit heute üblichem Luftaustausch würde nur zu unerwünschter Zugluft und gegebenenfalls zur Austrockung des Gleitmediums führen. Von daher wird die beschriebene Vorrichtung bevorzugt in einem Dampfbaderaum beispielsweise mit ansteigender Temperatur und ansteigender Luftfeuchtigkeit eingesetzt. Bei einer Zwei-Personen-Anordnung ist der Einsatz eines Klimadampfbadzeltes als äußerer Rahmen denkbar, der in geöffnetem Zustand als Ruhelager und in geschlossenem Zustand als Kräuterdampfbadumgebung eingesetzt werden kann.

Sollte eine einzelne Vorrichtung in einem allgemein klimatisierten Raum eingesetzt werden, so empfiehlt sich eine berührungsfreie Abdeckung mit einer Wärme reflektierenden Folie (beispielsweise mit Alubeschichtung), die mit einer Vorrichtung auf Distanz zum Körper gehalten werden kann, um das Gefühl von Zugluft, Kälte und die Austrockung des Mediums zu vermeiden.

Nach Beendigung der Anwendung kann zudem vor dem Verlassen der Vorrichtung durch den Badegast durch einen an einer Kabinendecke angeordneten Ventilator oder eine vergleichbare Einrichtung Wasser eingespritzt werden, welches durch die Fliehkraft im ganzen Raum verteilt wird und auch im Bereich der Vorrichtung und somit auf den Badegast herabspritzt.

Für den Fall, dass in dem betreffenden Raum eine Anlage zur Eisplättchen-Herstellung und/oder Verteilung vorgesehen ist, könnten diese Eisplättchen durch einen mit einem Ventilator verbundenen Plattenverteiler in den Raum eingestreut werden. Der Gast wird dadurch eine bisher kaum erlebte Erfrischung und Abkühlung des Körpers nach einem intensiv erwärmenden Gleitbad genießen. Das vom Ventilator an der Kabinendecke verteilte Wasser bewirkt zudem eine Reinigung des Badegastes und der Liegefläche.

Nur der Vollständigkeit halber wird erwähnt, dass zum Ein- und Aussteigen aus der Vorrichtung unterhalb der Folie in dem Vorrichtungs- oder Wannengrundkörper-Innenraum auch noch eine unterhalb der Folie 10 befindliche Hebe- und Senkeinrichtung vorgesehen sein kann.

Eine derartige Hebe- und Senkeinrichtung ist jedoch im Detail nicht gezeigt. Es wird insoweit auf bekannte Lösungen verwiesen.

Allerdings wird nachfolgend noch unter Bezugnahme auf Figur 2 eine weitere Abwandlung und Ergänzung gezeigt.

Bei dem Ausführungsbeispiel gemäß Figur 2 ist im Wanneninneren 1" eine auf dem Wannenboden ruhende oder in sonstiger Weise abgestützte Distanzeinrichtung 39 vorgesehen, beispielsweise in Form eines Distanzlochbleches 39'. Darunter befindet sich eine Heizeinrichtung 41. Bei eingelassenem Wasser oder anderem Medium wird nicht Luft, sondern dieses fließfähige Medium erwärmt, weil die Wärme auch durch die Öffnungen in der Distanzeinrichtung 39 in den oberhalb der Distanzeinrichtung 39 befindlichen Raum übergreift und sich hier ausbreitet.

Oberhalb der Distanzeinrichtung 39 ist in dem Ausführungsbeispiel gezeigt, dass hier nicht nur feststehende, sondern auch bewegliche Schwimmkörper, beispielsweise in Form von aufblasbaren Einsätzen 43 vorgesehen sein können, die sich in diesem Raum befinden, hier eingelegt sind und/oder an die Gleitfläche, d.h. die Gleitfolie 10 von unten her herangeklappt oder angehoben werden können. Sie können bei unkontrolliertem Bewegen eines Badegastes oberhalb der Gleitfolie einem unkontrolliertem Bewegungsvorgang entgegenwirken. Dadurch können bei manchen Badegästen auch eventuell entstehende Angstzustände vermieden werden. So spürt der Körper einen gewissen Gegendruck unterhalb der Folie, beispielsweise durch Verwendung eines schaumstoffförmigen Einsatzes, eines gepolsterten Einsatzes etc.. Auch in diesem Raum unterhalb der Folie vorgesehene schwimmende und/oder aufblasbare Körper, matratzenähnliche aufblasbare Körper, etc. können im Bereich Rücken, Gesäß, und/oder Knie unterstützend wirken. In Figur 2 sind die Einsätze 43 unten auf dem Distanzlochblech 39 ruhend gezeigt. Würde man aber nunmehr entsprechend ausreichendes Wasser, beispielsweise nach Wunsch entsprechend aufgewärmtes Wasser in die Wanne einfüllen (durch einen in Figur 2 nicht näher gezeigten Zulauf), würden mit dem Wasserstand auch der oder die Einsätze 43 mit steigen, bis sie je mit gewünschtem Anlagedruck an der Unterseite der Folie 10 anliegen.

In Figur 2 ist im rechtsliegenden Teil nur angedeutet, dass anstelle eines oder mehrerer beispielsweise schaumgummiähnlicher oder matratzenförmig aufblasbarer Schwimmkörper unterhalb der Folie auch eine Auftriebseinrichtung beispielsweise in Form einer schwimmfähigen Granulateinlage 43 mit einer Vielzahl von schwimmenden Granulat-Körnchen vorgesehen sein kann, die bei zugefülltem Wasser schwimmfähig ist und dann entsprechend den durch das Granulat erzeugten Auftriebskräften von der Unterseite her auf die Folie einwirken. Beliebige weitere Abwandlungen sind jederzeit möglich.

Bei der Querschnittsdarstellung gemäß Figur 3 ist alternativ dazu gezeigt, dass der Vorrichtung, d.h. dem Wanneninnenraum, zumindest ein Zuführrohr 45 und ein Ablassrohr 46 zugeordnet sein können, worüber beispielsweise ein fließfähiges Medium, beispielsweise Luft in den Wanneninnenraum zuströmen und durch das Ablassrohr 46 wiederum abströmen kann. Hierdurch kann beispielsweise auch durch eine außenliegende Heizung oder beispielsweise durch Verwendung einer Dampfheizung aufgewärmter oder erhitzter Dampf dem Innenraum zugeführt werden. Durch ständiges Umwälzen kann eine bewusste Temperaturaufrechterhaltung und/oder -veränderung realisiert werden. Genauso kann hier auch flüssiges und nicht nur gasförmiges Medium zugeführt werden.

Es ist bereits darauf hingewiesen worden, dass sich ein Gast, der sich anfänglich auf die Gleitfolie legt, aufgrund seines Eigengewichtes dazu beiträgt, dass die Gleitfolie 10 in einem gewissen Maße konkav durchhängt. Das Maß des Durchhängens kann jedoch durch diverse Maßnahmen begrenzt werden. Eine Begrenzung des Durchhängens der Gleitfolie 10 und des Eintauchens in die Vorrichtung oder in den sog. Wannengrundkörper kann von unten her auch durch unterschiedliche, dem Eintauchen entgegenwirkende Kräfte und Maßnahmen beeinflusst und gesteuert werden, zum Teil auch durch die verwendete Folie bzw. die verwendeten Materialien für die Folie selbst, die möglicherweise einen unterschiedlichen Grad eines Ausbauchens und Durchhängens erlaubt, weil beispielsweise ein elastisches oder zumindest geringfügig elastisches Material für die Folie verwendet wird. Durch all diese Maßnahmen wird zwar die Eintauchtiefe bestimmt und/oder verändert. Dabei wird jedoch eine "unweiche" Lagerung vermittelt. Von daher kann durch einen in der Wanne befindlichen verstellbaren Wannenboden und einer Schaumstoffauflage oder einer Schwebkörpereinlage in der Wanne im warmen Wasser schwimmend vorgesehen sein, um entsprechende Auftriebskräfte von der Unterseite der Folie her auf die Folie und damit auf die auf der Folienoberseite liegende Person einwirken zu lassen. Dies eröffnet auch die Möglichkeit einer verstellbaren Eintauch-Endpunktlagerung. Die Erwärmung und Warmhaltung des Bereichs unterhalb der Gleitfolie erfolgt auch in diesem Beispiel durch Beheizung des in dem Folienunterraum befindlichen Fluids, insbesondere Wasser, in welchem der Körper unter Zwischenschaltung der Folie eintaucht. Aber auch eine Warmluftheizung ist denkbar. In all den Fällen wird sichergestellt, dass der Temperaturverlauf dieses Mediums unterhalb der Folie steuer- und/oder veränderbar ist, wobei bevorzugt alle hierfür relevanten Parameter gemeinsam oder einzeln einstell- und veränderbar sind, um ein optimales Zusammenspiel der einzelnen Maßnahmen zu erreichen.

Ein auf der Folie befindliche Gast kann also durch die eigene Körperbewegung den Körper in eine rutschende und gleitende Bewegung auf der Folie bringen. Dieser Effekt kann gegebenenfalls noch dadurch verstärkt werden, dass beispielsweise über die Aufroll- und Zugeinrichtung 29 und /oder durch die Umlenkrollen 11 beispielsweise die Gleitfolie 10 immer um eine kurze Wegstrecke in die eine und dann wiederum in die andere Richtung bewegt werden, wodurch der Körper zusätzlich zum Gleiten angeregt wird. Diese Verschwenkung kann letztlich entgegen der Kraft der Federkraftspeicher 21 durchgeführt werden, beispielsweise durch gleichsinniges Verdrehen (vorzugsweise motorisch) durch die Umlenkrollen 11. Alternativ oder ergänzend kann zum Hin- und Herverfahren der Gleitfolie 10 aber auch die bereits erwähnte Aufroll- und Zugvorrichtung 29 verwendet werden, die im gezeigten Ausführungsbeispiel unterhalb der Tragvorrichtung 1, d.h. unterhalb des Bodens 7 der Tragvorrichtung 1 bzw. des Wannengrundkörpers 1' vorgesehen ist und zwei gegensinnig beispielsweise über einen Motor antreibbare Aufwickelrollen 31 umfasst. Über diese Aufwickelrollen wird eine folien- oder riemenförmige, seilförmige oder kettenförmige um Umlenkrollen 30 umlenkbare Zugeinrichtung 33 betätigt, die im gezeigten Ausführungsbeispiel an dem schienenförmigen Spannrahmen 19' an den beiden gegenüberliegenden Längsseiten 3 der Wanne 1' angreift.

Ergänzend kann diese gesteuerte Einwirkung zur Verstärkung des Gleiteffektes auch beispielsweise durch eine zusätzlich vorgesehene Kipp- oder Schwenkeinrichtung 151 für die gesamte Vorrichtung 1, d.h. im gezeigten Ausführungsbeispiel für den Wannengrundkörper 1', realisiert werden. Dies ist in Figur 2 schematisch angedeutet, indem auf der Wannenunterseite bei der dort wiedergegebenen Abstütz- und Standeinrichtung 153 zusätzlich eine in Längsrichtung der Vorrichtung verlaufende Schwenk- oder Kippachse 151 eingezeichnet ist. Dadurch kann beispielsweise eine Verschwenkung längs der Pfeildarstellung 155 bewirkt werden. Alternativ und ergänzend kann auch an der Vorrichtung eine quer dazu verlaufende weitere Schwenk- oder Kippachse ausgebildet sein, um eine Verschwenkung der gesamten Anlage und damit der Folie um eine quer zur Längsrichtung verlaufende Achse zu ermöglichen. Wird beispielsweise die Vorrichtung über eine Längs- und eine Querachse gleichzeitig bewegt, gegebenenfalls auch durch eine überlagerte Schwingung und gegebenenfalls sogar mit unterschiedlich langer Schwingungsdauer, so wird eine räumliche Schwenkbewegung der Folie initiiert, wodurch eine darauf befindliche Person leichter in eine gleitende Körperbewegung gegenüber der sie tragenden Gleitfolie gelangt.

Umgekehrt kann auch eine Einrichtung vorgesehen sein, beispielsweise unter Verwendung von Rollen, die es ermöglichen, durch einen Bademeister oder motorisch beispielsweise die gesamte Vorrichtung im Sinne einer translatorischen Bewegung auf den Rollen hin und her zu verstellen, um die Gleitbewegung auf der Folie 10 zu unterstützen und zu verstärken. Genauso könnte die Wanne auch möglicherweise um eine vertikale oder sonstig geneigte Achse eine Drehbewegung durchführen, um die Gleitbewegung zu verstärken. Ausreichen würde auch, wenn lediglich die Umlenkeinrichtung 11 bzw. die Umlenkrollen 11' eine entsprechende Rotations-Verschwenk- oder Kipp- oder translatorische Bewegung relativ zur Vorrichtung oder zum Wannengrundkörper 1' durchführen könnten, um die gleichen Effekte zu erzielen. In allen Fällen ergibt sich dadurch eine Relativbewegung verbunden mit einer Schwerpunktverlagerung der auf der Folie befindlichen Person.

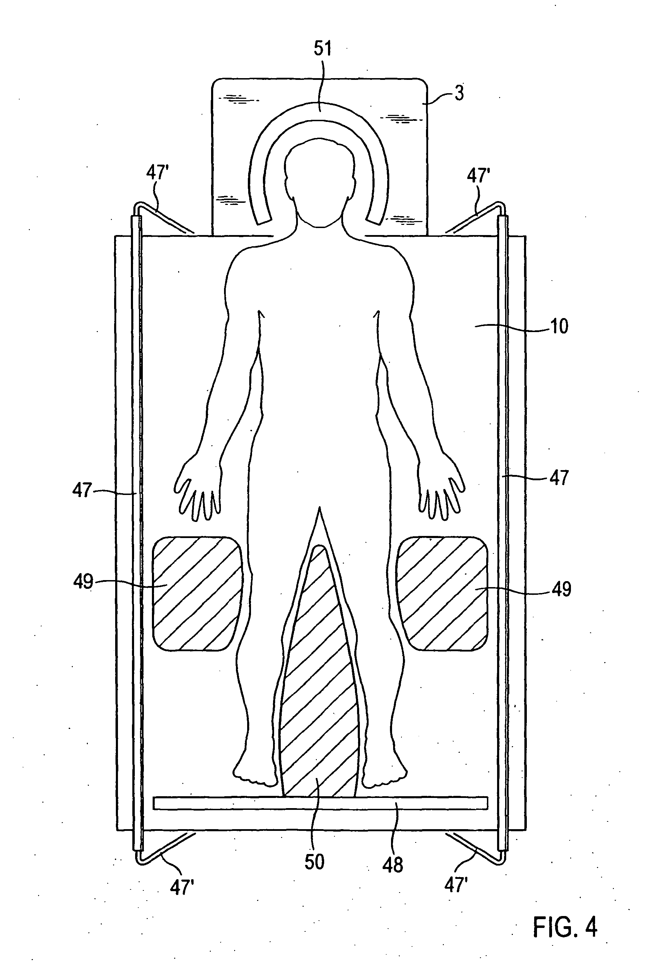

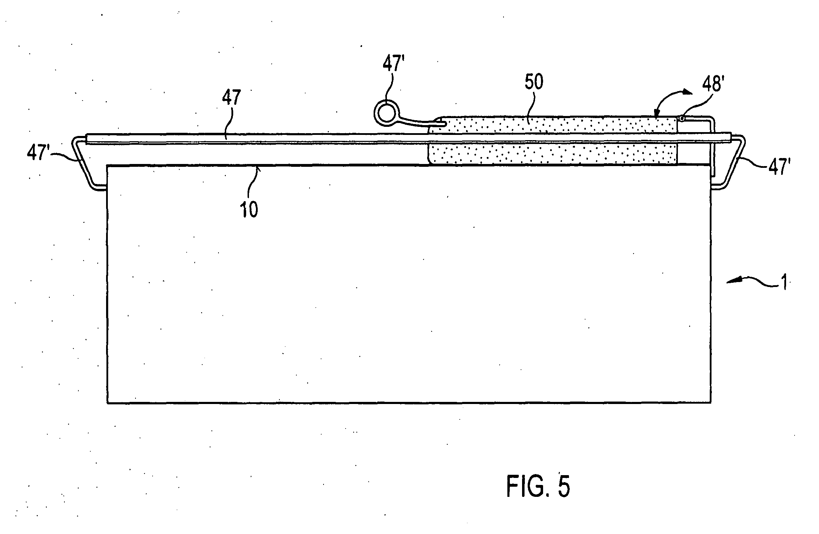

Anhand von Figur 4 ist schließlich auch noch gezeigt, dass beispielsweise dann, wenn sich ein Gast auf der Folie zur Anwendung befindet, von außen her über den Randbereich 9 der Vorrichtung und insbesondere über die Umlenkeinrichtung 11 hinweg von außen her seitliche Halte- und Greifeinrichtungen 47 umgeklappt werden können, die es dem Badenden erleichtert, den Körper gleitend auf der kuhlenförmigen Gleitfolie 10 zu bewegen und um ihm andererseits auch mehr Sicherheit zu vermitteln. In Figur 4 ist in Draufsicht und in Figur 5 in Seitenansicht dabei benachbart zu den seitlichen Umlenkrollen 11' jeweils eine fast über die gesamte Länge der Vorrichtung verlaufende Haltestange 47 gezeigt, die über endseitige Schwenkhebel 47' von einer außerhalb der Vorrichtung befindlichen Außereingriff-Position bis über den Randbereich der Vorrichtung nach innen schwenkbar und in dieser Stellung arretierbar sind.

Ein das eigene Wohlbefinden steigernder Effekt kann auch dadurch erzielt werden, dass zur Eindämmung einer unkontrollierten Gleitbewegung nicht nur seitliche Stützkeile 49 zur Abstützung, sondern beispielsweise auch noch ein gepolstertes oder weiches Innenteil 50 zwischen den Beinen positioniert werden kann. Diese Teile können beispielsweise ebenfalls durch Schwenkachsen von außen nach innen eingeschwenkt werden, dass sie zumindest in gewissem Maße positionierbar sind und nicht frei lageveränderlich sind. So kann der in den Figuren 4 und 5 gezeigte Innenkeil 50 beispielsweise um eine als Fußauflage oder Abstützung 48 dienende Achse 48' von einer nach außen geschwenkten Position bis auf zur Auflage auf die Gleitfolie geschwenkt werden. Dadurch wird zum einen der Körper etwas stabilisiert. Zum anderen kann durch Auswärtschwenken dieses gepolsterten Innenteils die Möglichkeit geschaffen, dass sich der Badende vom Rücken auf die Bauchlage und umgekehrt dreht. Anschließend kann dieser Keil wieder auf die Gleitfolie 10 zurückgeschwenkt werden.

Zusätzlich können an diesem Innenteil wie aber auch an dem seitlichen Stützkeil 49 ergänzende Traggriffe 47' ausgebildet sein, wie dies auch in der Seitendarstellung gemäß Figur 5 nur schematisch angedeutet ist.

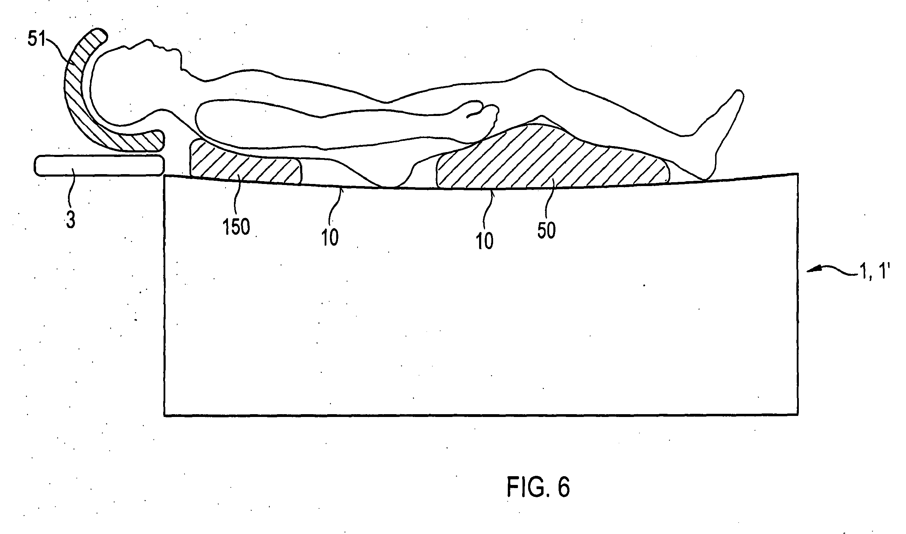

Schließlich zeigt Figur 6 nicht nur das als Kniekeil ausgebildete Innenteil 50, wodurch das Knie etwas höher gelegt und die Beine angewinkelt werden können, sondern zeigt auch die Verwendung eines Rückenkeiles 150. Durch die Verwendung der vorstehend genannten Unterlegeinsätze 50, 150 können beispielsweise zielgerichtet der Schulterbereich und der Rücken und teilweise auch der Bein- und Kniebereich etwas mehr angehoben werden, so dass nunmehr das Hauptgewicht im Beckenbereich auf der Gleitfolie ruht. An dieser, nunmehr tiefsten Stelle der Gleitfolie kann zusätzliches Medium eingebracht werden, so dass hier zumindest ansatzweise ein Teilbad entsteht, falls dies gewünscht wird.

Alternativ könnten die erwähnten Innenteile 50 auch mit der Gleitfolie fest verbunden sein, um hier den gleitfähigen freien Raum zumindest etwas zu begrenzen. Alternativ können die entsprechenden Einlegeteile 50 auch in anderer Weise fixiert sein, ohne mit der Gleitfolie 10 fest verbunden sein zu müssen.

Lediglich in Figur 4 und 5 ist noch ergänzend angedeutet, dass sich der Kopf eines die Anwendung durchführenden Gastes bevorzugt außerhalb der Tragvorrichtung, d.h. des Wannengrundkörpers 1' befindet, beispielsweise in einer bevorzugt isolierenden Kopfschale 51 eingelegt sein kann, um eine unerwünschte Bewegung des Kopfes zu verhindern. Hierüber wird auch der Körper stabilisiert. Dadurch kann der Körper jedenfalls nicht unkontrolliert aus dem Bereich Folie herausgleiten. Wird die Kopf- und Nackenstütze jedoch lose am Nacken des Badegastes fixiert, dann können alle Gleitbewegungen durchgeführt werden. Kopf und Nacken bleiben gepolstert und von einer unerwünschten Erwärmung gleichwohl ausgenommen. In diesem Falle ist bevorzugt nur eine allgemein als Kopfauflage bezeichnete Abstützeinrichtung 3 vorgesehen, wie sie in Figur 1 und 4 nur angedeutet ist.

Wie aus der Schilderung hervorgeht, erstreckt sich die Folie in ausreichendem Maße in Längsrichtung der Vorrichtung oder Wanne. Insbesondere wenn auf der Folienunterseite kein wässriges Fluid eingegeben ist, sondern sich lediglich Luft, erhitzte Luft oder Wasserdampf befinden, muss die Folie in ihrer Längsrichtung nur so bemessen sein, dass sie den Körper insgesamt aufnehmen kann. Bevorzugt sind aber stirnseitige verlängerte Abschnitte vorgesehen, müssen allerdings nicht vorgesehen sein. Es kann zumindest an einer Stirnseite der Vorrichtung dann eine entsprechende Kopfauflage vorgesehen sein. Die Kopfauflage kann aber mit einer entsprechenden Wärmeisolierung auch innerhalb der Vorrichtung oder Wanne angeordnet sein. Bevorzugt ist sie ebenfalls elastisch und/oder nachgiebig, so dass sich hier am Aufliegen an der Folie keine als unangenehm empfundene Kante ergibt.

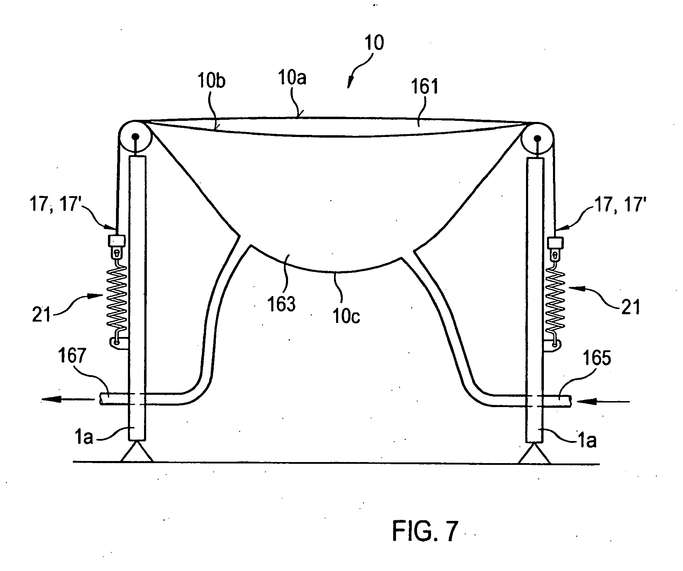

Die Gleitfolie 10 kann insbesondere durch vielmaliges Benutzen an bestimmten Stellen gedehnt worden sein. Dies könnte zur Folge haben, dass in der horizontalen Ausgangsstellung der Folie diese nunmehr gleichwohl bereits in der Ausgangsstellung doch etwas stärker durchhängt, wodurch sich beim Auftragen von Gleitmedien diese in die Mitte zusammenfließen würden. Dies könnte dadurch vermieden werden, dass beispielsweise, wie in der Querschnittsdarstellung gemäß Figur 7 angedeutet ist, die Folie doppelwandig als Hohlkörpersystem ausgebildet ist, wobei die obere Deckfolie 10a und die untere Deckfolie 10b unter Ausbildung eines Folienhohlraumes 161 an den Rändern 17 verschweißt ist. Ist ferner eine Ein- und/oder Auslaßöffnung vorgesehen, kann hierüber ein Medium eingepumpt werden, welches durch eine Wölbung nach oben hin das Durchhängen der Folie ausgleicht. Wird nunmehr für die obere Folie 10a eine Folie mit besonderen Gleiteigenschaften und einem weichen Griff und eine untere Folie 10b beispielsweise mit gewebeverstärkten Materialien verwendet, die nunmehr vor allem die gesamte Tragkraft aufnehmen soll, so erreicht man dadurch eine Stützung der oberen Gleitfolie 10a.

Im Übrigen könnte durch die erwähnte Befüllungsöffnung je nachdem geeignetes gas- oder vorzugsweise fließfähiges Medium, auch dick pastöses oder gelartiges Medium in den Hohlraum 161 eingepumpt werden, und zwar in einem derartigen Maße, dass die obere Fläche, d.h. die Gleitfolie 10a selbst bei einem aufgelegten Badegast praktisch mehr oder weniger fast horizontal ausgerichtet ist und bleibt oder nur eine geringfügige konkave Vertiefung aufweist, wobei der Grad der Durchwölbung durch das zugepumpte Medium, welches sich an der unteren Tragfolie 10b abstützt, einstellbar ist.

Eine solche doppelte Folie mit zumindest zwei Folienschichten, die aufeinander verschweißt, verklebt oder unter Ausbildung des erwähnten Folienhohlraumes 161 gebildet sein können, erlaubt es, eine möglichst gleitfähige Folienoberschicht und eine demgegenüber ausreichend dimensionierte oder mit Gewebestoffen versehene untere Folien-Tragschicht auszubilden.

Von daher kann auch eine ausreichende Tragkräfte aufnehmende bevorzugt gewebearmierte Folie vorgesehen sein, die an ihrer Oberfläche mit ihrer besonders gut gleitenden Schicht als Folienoberseite 10a ausgestattet ist. In diesem Falle wäre also kein Hohlraum vorgesehen.

Wie aber anhand von Figur 5 gezeigt ist, ist bevorzugt noch eine dritte Folie 10c oder Folienschicht vorgesehen, die unterhalb der eigentlichen gewerbeverstärkten Tragfolie 10b vorgesehen ist und von den seitlichen Umlenkrollen bauchig tiefer in die Vorrichtung hinabhängt. Auch diese dritte Folie oder Folienschicht 10c ist mit den anderen Folienschichten vorzugsweise am Randbereich 17, 17' dicht verbunden, z.B. verschweißt, so dass sich hierdurch ein weiterer geschlossener Hohlraum 163 ergibt. Diese dritte Folie 10c ist bevorzugt ebenfalls wieder gewebeverstärkt, so dass bei einem Bruch der oberen gewebeverstärkten Folie oder Folienschicht 10b zumindest diese tiefer herabhängende Folie einen Badenden noch auffangen kann.

Der untere Hohlraum 163 ist zumindest mit einer Zuführöffnung, vorzugsweise einer Zulauf- oder einer Ablauföffnung 165, 167 versehen, wo beispielsweise beheizbare Luft zugeführt werden kann. Dadurch kann das gesamte System aufgewärmt oder auf eine bestimmte Temperatur gebracht werden, da die Temperatur dann über den oberen Folien 10b und 10a und das gegebenenfalls dazwischen befindliche Ausgleichsmedium bis zum Badegast aufsteigt. Ist in dem Rücklauf ein einstellbares Drosselventil vorgesehen oder ist die Abströmöffnung kleiner gestaltet, so wird in der unteren Aufwärmkammer 163 stets ein gewisser Überdruck gewährleistet.

Durch entsprechende Druckeinstellung kann dadurch auch die Horizontallage der obersten Gleitschicht 10a entsprechend eingestellt werden. Zur Reinigung der gesamten Vorrichtung kann der Überdruck sogar erhöht werden, dass die oberste Auflagefläche 10' der Gleitfolie 10a sogar zumindest leicht konvex aufbaucht, so dass das Reinigungsmittel und die Reinigungsflüssigkeit über die Folie seitlich ablaufen kann.

In der oberen Kammer 161 kann auch bevorzugt nicht nur gasförmiges der fließfähiges, sondern auch pastöses oder dick pastöses oder gelförmiges Medium eingeführt werden, wodurch unter Umständen interessante Effekte erzielbar sind. Insbesondere bei Verwendung einer dritten unteren Folie 10c, deren darüber befindlicher Hohlraum 163 durch Zupumpen eines flüssigen oder vorzugsweise gasförmigen Mediums (zur Gewichtsreduzierung) unterschiedlich druckbelastbar und befüllbar ist, kann auch vorgesehen sein, dass der Hohlraum 161 zwischen der obersten Gleitfolie 10a und der darunter befindlichen ersten Tragfolie 10b dauerhaft fest verschlossen ist. Durch die Gasbefüllung im Unterraum 163 kann ebenfalls wieder der Grad des Durchhängens oder der Horizontalausrichtung der zuoberst liegenden Gleitfolie 10a eingestellt werden. Zur Reinigung kann der Hohlraum 163 so aufgebläht werden, dass sich eine konvexe Krümmung an der oberen Folie 10a einstellt, so dass Reinigungswasser über die Folie seitlich abläuft.

Durch die verwendete dritte, d.h. unterste Folie 10c und der dadurch gebildeten Kammer 163, die als Aufwärmkammer dient, kann letztlich auf einen Wannengrundkörper 1' völlig verzichtet werden. Es genügt in der Tat eine einfache Tragvorrichtung 1, die beispielsweise auf vier Füßen 1a aufgestellt sein kann, wobei an dem oberen bevorzugt umlaufenden Rahmen dann die zumindest gegenüberliegenden seitlichen Auflageränder 9 und/oder die dort positionierten Umlenkrollen 11' vorgesehen sind, um die Folie 10 zu tragen und abzustützen.

## Patentansprüche

1. Vorrichtung zur Durchführung einer Anwendung, nämlich unter Verwendung von Gleitmedien in Form von flüssigen, fließfähigen, pastösen, cremigen, gleitenden und/oder rollenden Zusatzstoffen, mit folgenden Merkmalen:
- mit einer rahmenförmigen Trageinrichtung (1), vorzugsweise in Form eines Wannengrundkörpers (1'),
- an der Tragvorrichtung (1) ist eine Folie (10) gehalten,
- die Folie (10) ist zumindest abschnittsweise und/oder gegenüberliegend an einem umlaufenden Rand (9) der Tragvorrichtung (1) gehalten und/oder abgestützt,
- die Folie (10) hängt während der Anwendung gegenüber einer Horizontalebene (E) durch,
**gekennzeichnet durch** die folgenden weiteren Merkmale:
- die Folie (10) befindet sich zur Durchführung der Anwendung in eine Position oder ist zur Durchführung der Anwendung in eine Position bringbar, in welcher die seitlichen Abschnitte der Folie (10), die zu ihrer seitlichen Randabstützung führen, gegenüber einer Horizontalebene (E) in einem Winkel (α) ansteigen, der kleiner als 45° ist,
- die Vorrichtung ist so gestaltetet, dass die Folie (10) eine Auflagefläche bildet, die schalen-, teller- oder wokähnlich gestaltet ist, und
- die Folie (10) ist als Gleitfolie so gestaltet und beschaffen, dass deren Oberseite zumindest unter zusätzlicher Verwendung von Anwendungs-und/oder Gleitmedium gleitfähig ist.

2. Vorrichtung nach Anspruch 1, **dadurch gekennzeichnet, dass** bei einer aufgelegten Person (27) die seitlich von der Person zu den Randabschnitten führenden Folienabschnitte in einem Winkel (α) kleiner als 40°, insbesondere kleiner als 35°, 30° und/oder 25° ansteigen.

3. Vorrichtung nach Anspruch 1 oder 2, **dadurch gekennzeichnet, dass** die Folie (10) an zumindest zwei gegenüberliegenden Seiten (3) der Tragvorrichtung (1) zumindest abschnittsweise gehalten sind.

4. Vorrichtung nach Anspruch 3, **dadurch gekennzeichnet, dass** die Folie (10) an zumindest zwei gegenüberliegenden Seiten (3) der Tragvorrichtung (1) mittels einer Umlenkeinrichtung (11) gehalten und/oder geführt ist.

5. Vorrichtung nach Anspruch 3 oder 4, **dadurch gekennzeichnet, dass** die Folie (10) außerhalb der Tragvorrichtung (1) über eine Halte- und/oder Verankerungseinrichtung (19) gehalten ist.

6. Vorrichtung nach Anspruch 5, **dadurch gekennzeichnet, dass** die Halte- und/oder Verankerungsvorrichtung (19) für die Folie (10) eine Spannvorrichtung (21), vorzugsweise in Form eines Federkraftspeichers (21") umfasst.

7. Vorrichtung nach einem der Ansprüche 1 bis 6, **dadurch gekennzeichnet, dass** die Folie (10) eine Gewebeverstärkung umfasst oder mit einer Gewebeverstärkung unterlegt ist.

8. Vorrichtung nach einem der Ansprüche 1 bis 7, **dadurch gekennzeichnet, dass** die Folie (10) mit einer gut gleitfähigen Auflagenfläche (10') versehen ist.

9. Vorrichtung nach einem der Ansprüche 1 bis 8. **dadurch gekennzeichnet, dass** die Folie (10) zumindest zweilagig ausgebildet ist und eine gut gleitende zuoberst liegende Folie (10a) und eine darunter befindliche Tragfolie (10b) umfasst, die vorzugsweise eine Gewebeverstärkung aufweist.

10. Vorrichtung nach Anspruch 8, **dadurch gekennzeichnet, dass** die zuoberst liegende Folie (10a) und die darunter befindliche Folie (10b) unter Ausbildung eines geschlossenen und dichten Hohlraumes (161) in einem Umfangsbereich fest miteinander verbunden sind, d.h. vorzugsweise an ihrem umlaufenden Randbereich (17, 17') vernäht, verschweißt, verklebt oder vulkanisiert sind.

11. Vorrichtung nach Anspruch 9 oder 10, **dadurch gekennzeichnet, dass** zwischen der gleitfähigen zuoberst liegenden Folie (10a) und der ausreichende Tragkräfte aufnehmenden darunter befindlichen Folie (10b ein Hohlraum (161) gebildet ist, der mit einem Medium, vorzugsweise einem gasförmigen, fließfähigen, pastösen und/oder gelförmigen Medium befüllt oder über eine vorzugsweise verschließbare Befüllungsöffnung befüllbar ist.

12. Vorrichtung nach einem der Ansprüche 9 bis 11, **dadurch gekennzeichnet, dass** der Hohlraum (161) mit einem Medium so befüllt oder vorzugsweise über eine verschließbare Zulauföffnung befüll- und bei Bedarf ganz oder teilweise so entleerbar ist, dass durch die leicht durchhängende Tragfunktion aufnehmende Folie (10b) und dem Befüllungsgrad in dem Hohlraum (161) die zuoberst liegende Gleitfolie (10, 10a) in ihrer Lage einstellbar ist, vorzugsweise horizontal, quasi horizontal oder mit einer vorwählbaren geringen konkaven Durchbiegung zu liegen kommt.

13. Vorrichtung nach einem der Ansprüche 1 bis 12, **dadurch gekennzeichnet, dass** eine weitere Folie (10c) vorgesehen ist, die unterhalb der Tragfunktion erfüllenden Folie (10b) angeordnet ist.

14. Vorrichtung nach Anspruch 13, **dadurch gekennzeichnet, dass** die untere Folie (10c) mit der Folie (10b) und vorzugsweise auch mit der Folie (10a) in einem Außenumfang, vorzugsweise an ihrem Rand (17, 17') unter Ausbildung eines Hohlraumes (163) fest verbunden, vernäht, verschweißt, verklebt etc. ist.

15. Vorrichtung nach Anspruch 13 oder 14, **dadurch gekennzeichnet, dass** der Hohlraum (163) mit einem Wärmemedium befüllbar und/oder durch ein umströmendes Wärmemedium beheizbar ist.

16. Vorrichtung nach Anspruch 15, **dadurch gekennzeichnet, dass** die nach unten durchhängende Folie (10c) ebenfalls Tragfunktion vorzugsweise unter Verwendung einer Gewebeverstärkung erfüllt.

17. Vorrichtung nach einem der Ansprüche 1 bis 16, **dadurch gekennzeichnet, dass** die Folie (10) vorzugsweise mit den Einzelfolien (10a, 10b, 10c) durch eine mit einer Stand- oder Fußeinrichtung versehenen Tragvorrichtung (1) gehalten ist.

18. Vorrichtung nach einem der Ansprüche 1 bis 17, **dadurch gekennzeichnet, dass** eine Hebe- und Senkeinrichtung für die Folie (10) vorgesehen ist.

19. Vorrichtung nach einem der Ansprüche 1 bis 18, **dadurch gekennzeichnet, dass** die Folie (10) bei Auflage einer Person im Querschnitt eine leichte schalen- oder wokähnliche Form einnimmt.

20. Vorrichtung nach einem der Ansprüche 1 bis 19, **dadurch gekennzeichnet, dass** im Folienunterraum (1") Einsätze (43) vorhanden sind, die mit der Folienunterseite wechselwirken.

21. Vorrichtung nach Anspruch 20, **dadurch gekennzeichnet, dass** die Einsätze (43) aus elastischen und/oder verformbaren Materialien bestehen.

22. Vorrichtung nach Anspruch 21, **dadurch gekennzeichnet, dass** der zumindest eine Einsatz (43) aus Schaumgummi oder Schaumstoff oder vergleichbarem Material besteht, so dass eine auf der Folie (10) befindliche Person von der Oberseite auf den so gebildeten Einsatz (43) vorzugsweise unter zumindest geringfügiger Kompression dieses Einsatzes (43) aufliegt.

23. Vorrichtung nach Anspruch 20, **dadurch gekennzeichnet, dass** der Einsatz (43) aus vorzugsweise schwimmfähigem Granulat besteht.

24. Vorrichtung nach einem der Ansprüche 1 bis 20, **dadurch gekennzeichnet, dass** die Tragvorrichtung (1) einen Wannengrundkörper (1') umfasst, und dass der Raum zwischen der Unterseite der Folie (10) und dem Wanneninnenraum (1") mit flüssigem und/oder gasförmigem Medium beströmbar ist, vorzugsweise in Form einer Umwälzeinrichtung unter Verwendung zumindest eines Zulauf- und zumindest eines Ablaufrohres (45, 46).

25. Vorrichtung nach einem der Ansprüche 1 bis 24, **dadurch gekennzeichnet, dass** zumindest zwei seitliche Halte- und/oder Greifeinrichtungen (47) von außen her zumindest in den Randbereich der Folie (10) bringbar, verschwenkbar und/oder umlegbar und vorzugsweise in dieser Position fixierbar sind.

26. Vorrichtung nach einem der Ansprüche 1 bis 25, **dadurch gekennzeichnet, dass** auf der Folie (10) von dieser getrennt seitliche, vorzugsweise gepolsterte Abstützeinrichtung (49) und/oder ein vorzugsweise elastisches, weiches und/ oder gepolstertes Innenteil (50) und/oder Knieauflageteil (50) vorgesehen ist.

27. Vorrichtung nach Anspruch 16, **dadurch gekennzeichnet, dass** die zumindest eine Abstützeinrichtung (49) und/oder ein Innen- oder Knieauflageteil (50) von einer auf der Gleitfolie (10) befindlichen Einsatzposition in eine Außereingriffposition nach außen verschwenkbar sind.

28. Vorrichtung nach Anspruch 26 oder 27, **dadurch gekennzeichnet, dass** das Innen- und/oder Knieauflageteil (50) vorzugsweise um eine Fußabstützung (48, 48') verschwenkbar ist.

29. Vorrichtung nach einem der Ansprüche 26 bis 28, **dadurch gekennzeichnet, dass** die zumindest eine Abstützeinrichtung (49) und/oder das Innen- und/oder Knieauflageteil (50) mit Greifeinrichtungen (47') versehen sind.

30. Vorrichtung nach einem der Ansprüche 1 bis 29, **dadurch gekennzeichnet, dass** eine manuell und/oder motorisch zumindest die Halte- und Umlenkeinrichtung der Folie (10) und vorzugsweise der gesamten Tragvorrichtung (1) bzw. des Wannengrundkörpers (1') in Bewegung versetzende Einrichtung vorgesehen ist.

31. Vorrichtung nach Anspruch 30, **dadurch gekennzeichnet, dass** die Einrichtung eine translatorische Bewegung, eine Kipp- oder Schwenkbewegung und/oder zumindest eine Rotationsbewegung zumindest der Gleitfolie (10), d.h. vorzugsweise einschließlich der sie tragenden Umlenkeinrichtung (11) ermöglicht.

32. Vorrichtung nach Anspruch 31, **dadurch gekennzeichnet, dass** die entsprechende Einrichtung unterhalb der Tragvorrichtung (1) oder des Wannengrundkörpers (1') vorgesehen ist.

33. Vorrichtung nach einem der Ansprüche 14 bis 16, **dadurch gekennzeichnet, dass**
der Hohlraum (163) so aufblähbar ist, dass die oberste Folie (10a) zur Ermöglichung des seitlichen Ablaufens von Reinigungswasser eine konvexe Krümmung einnimmt.

## Claims

1. Device for carrying out a treatment, namely using lubricants in the form of liquid, free-flowing, paste-like, creamy, sliding and/or rolling additives, comprising the following features:
- comprising a frame-like carrying means (1), preferably in the form of a tub base body (1'),
- a sheet (10) is held on the carrying device (1),
- the sheet (10) is held and/or supported at least in portions and/or on opposing sides on a peripheral edge (9) of the carrying device (1),
- during the treatment the sheet (10) sags relative to a horizontal plane (E),
**characterised by** the following further features:
- for carrying out the treatment the sheet (10) is located in a position, or for carrying out the treatment can be brought into a position, in which the lateral portions of the sheet (10), which lead to the lateral edge support thereof, ascend relative to a horizontal plane (E) at an angle (α) which is smaller than 45°,
- the device is configured in such a way that the sheet (10) forms a contact surface which is configured in a bowl-, dish- or wok-like manner, and
- the sheet (10) is configured and provided as a sliding sheet (10), such that its upper side is slidable at least with additional use of treatment medium and/or lubricant.

2. Device according to claim 1, **characterised in that** when a person (27) lies down on the device the sheet portions leading laterally from the person to the edge portions ascend at an angle (α) smaller than 40°, in particular smaller than 35°, 30° and/or 25°.

3. Device according to either claim 1 or claim 2, **characterised in that** the sheet (10) is held at least in portions on at least two opposing sides (3) of the carrying device (1).

4. Device according to claim 3, **characterised in that** the sheet (10) is held and/or guided on at least two opposing sides (3) of the carrying device (1) by means of a deflecting means (11).

5. Device according to either claim 3 or claim 4, **characterised in that** the sheet (10) is held outside the carrying device (1) via a holding and/or anchoring means (19).

6. Device according to claim 5, **characterised in that** the holding and/or anchoring means (19) for the sheet (10) comprises a tensioning device (21), preferably in the form of a spring energy store (21").

7. Device according to any one of claims 1 to 6, **characterised in that** the sheet (10) comprises a fabric reinforcement or is underlaid with a fabric reinforcement.

8. Device according to any one of claims 1 to 7, **characterised in that** the sheet (10) is provided with a highly slidable contact surface (10').

9. Device according to any one of claims 1 to 8, **characterised in that** the sheet (10) has at least two layers and comprises a highly slidable uppermost sheet (10a) and a carrying sheet (10b) located thereunder, which preferably comprises a fabric reinforcement.

10. Device according to claim 8, **characterised in that** the uppermost sheet (10a) and the sheet (10b) located thereunder are rigidly connected to one another in a peripheral region, i.e. are preferably sewn, bonded, glued or vulcanised to one another on their peripheral edge region (17, 17'), thus forming a closed and sealed hollow space (161).

11. Device according to either claim 9 or claim 10, **characterised in that** a hollow space (161) is formed between the slidable uppermost sheet (10a) and the sheet (10b) which is located thereunder and absorbs sufficient carrying forces, which hollow space is filled with a medium, preferably a gaseous, free-flowing, paste-like and/or gel-like medium, or can be filled therewith via a preferably closable filling aperture.

12. Device according to any one of claims 9 to 11, **characterised in that** the hollow space (161) is filled, or preferably can be filled with a medium and, if necessary, wholly or partially emptied via a closable supply aperture, such that the uppermost sliding sheet (10, 10a) can be adjusted in position via the slightly sagging sheet (10b) which assumes the carrying function and the filling level in the hollow space (161), and preferably comes to rest horizontally, quasi-horizontally or with a preselectable slight concave deflection.

13. Device according to any one of claims 1 to 12, **characterised in that** a further sheet (10c) is provided, which is arranged below the sheet (10b) which performs the carrying function.

14. Device according to claim 13, **characterised in that** the lower sheet (10c) is firmly connected, sewn, bonded, glued, etc. to the sheet (10b) and preferably also to the sheet (10a) in an outer periphery, preferably on its edge (17, 17'), thus forming a hollow space (163).

15. Device according to either claim 13 or claim 14, **characterised in that** the hollow space (163) can be filled with a heating medium and/or can be heated via a circulating heating medium.

16. Device according to claim 15, **characterised in that** the downwards sagging sheet (10c) also performs a carrying function, preferably using a fabric reinforcement.

17. Device according to any one of claims 1 to 16, **characterised in that** the sheet (10) preferably comprising the individual sheets (10a, 10b, 10c) is held via a carrying device provided with a stand means or foot means.

18. Device according to any one of claims 1 to 17, **characterised in that** a raising and lowering means is provided for the sheet (10).

19. Device according to any one of claims 1 to 18, **characterised in that** the sheet (10) assumes a slight bowl- or wok-like shape in cross-section when a person lies down on the device.

20. Device according to any one of claims 1 to 19, **characterised in that** inserts (43) are present in the space (1") below the sheet, which interact with the underside of the sheet.

21. Device according to claim 20, **characterised in that** the inserts (43) consist of resilient and/or deformable materials.

22. Device according to claim 21, **characterised in that** the at least one insert (43) consists of foam rubber or foam or a comparable material, so that a person located on the sheet (10) lies from the upper side onto the insert (43) thus formed, preferably at least slightly compressing this insert (43).

23. Device according to claim 20, **characterised in that** the insert (43) consists of preferably floatable granular material.

24. Device according to any one of claims 1 to 20, **characterised in that** the carrying device (1) comprises a tub base body (1') and **in that** a liquid and/or gaseous medium can flow through the space between the underside of the sheet (10) and the interior of the tub (1"), preferably in the form of a circulation device using at least one supply tube and at least one discharge tube (45, 46).

25. Device according to any one of claims 1 to 24, **characterised in that** at least two lateral holding and/or gripping means (47) can be brought, pivoted and/or transferred from the outside at least into the edge region of the sheet (10) and can preferably be fixed in this position.

26. Device according to any one of claims 1 to 25, **characterised in that** lateral, preferably padded support means (49) and/or a preferably resilient, flexible and/or padded inner part (50) and/or knee rest part (50) are provided on the sheet (10), being separated therefrom.

27. Device according to claim 16, **characterised in that** the at least one support means (49) and/or an inner or knee rest part (50) can be pivoted outwards from an in-use position located on the sliding sheet (10) into a disengaged position.

28. Device according to either claim 26 or claim 27, **characterised in that** the inner and/or knee rest part (50) can preferably be pivoted about a foot support (48, 48').

29. Device according to any one of claims 26 to 28, **characterised in that** the at least one support means (49) and/or the inner and/or knee rest part (50) are provided with gripping means (47').

30. Device according to any one of claims 1 to 29, **characterised in that** a means is provided which, manually and/or in a motor-driven manner, sets in movement at least the holding and deflecting means of the sheet (10) and preferably the entire carrying device (1) and the tub base body (1').

31. Device according to claim 30, **characterised in that** the means makes possible a translatory movement, a tilting or swivelling movement and/or at least a rotational movement of at least the sliding sheet (10), i.e. preferably including the deflecting means (11) carrying said sheet.

32. Device according to claim 31, **characterised in that** the corresponding means is provided below the carrying device (1) or the tub base body (1').

33. Device according to any one of claims 14 to 16, **characterised in that** the hollow space (163) can be inflated in such a way that the uppermost sheet (10a) assumes a convex curvature to allow cleaning water to run off laterally.

## Revendications

1. Dispositif pour l'exécution d'une application, à savoir en utilisant des milieux coulissants sous la forme de produits additionnels liquides, fluides, pâteux, crémeux, glissants et/ou roulants, avec les éléments techniques suivants :
- un dispositif porteur (1) en forme de cadre, de préférence sous la forme d'un corps de base en cuvette (1'),
- une feuille (10) est tenue sur le dispositif porteur (1),
- la feuille (10) est tenue et/ou soutenue au moins par tronçons et/ou à l'opposé d'une bordure périphérique (9) du dispositif porteur (1),
- la feuille (10) est en suspension pendant l'application par rapport à un plan horizontal (E),
**caractérisé par** les autres éléments techniques suivants :
- la feuille (10) se trouve, pour l'exécution de l'application, dans une position ou peut être amenée dans une position dans laquelle les tronçons latéraux de la feuille (10), qui mènent à son soutien en bordure latérale, montent par rapport à un plan horizontal (E) sous un angle (α) qui est inférieur à 45°,
- le dispositif est conçu de telle façon que la feuille (10) forme une surface de pose, qui est conçue à la manière d'une coque, d'une assiette ou d'une poêle dite "wok", et
- la feuille (10) est conçue et réalisée, à titre de feuille de coulissement, de sorte que sa face supérieure soit coulissante au moins en utilisant en supplément un milieu d'application et/ou de coulissement.

2. Dispositif selon la revendication 1, **caractérisé en ce que** lorsqu'une personne (27) est posée, les tronçons de feuille qui mènent vers les tronçons de bordure latéralement par rapport à la personne, montent sous un angle (α) inférieur à 40°, en particulier inférieur à 35°, 30° et/ou 25°.

3. Dispositif selon la revendication 1 ou 2, **caractérisé en ce que** la feuille (10) est tenue au moins par tronçons sur au moins deux côtés opposés (3) du dispositif porteur (1).

4. Dispositif selon la revendication 3, **caractérisé en ce que** la feuille (10) est tenue et/ou guidée au moyen d'un système de renvoi (11) sur au moins deux côtés opposés (3) du dispositif porteur (1).

5. Dispositif selon la revendication 3 ou 4, **caractérisé en ce que** la feuille (10) est maintenue, à l'extérieur du dispositif porteur (1), via un système de maintien et/ou d'ancrage (19).

6. Dispositif selon la revendication 5, **caractérisé en ce que** le dispositif de maintien et/ou d'ancrage (19) comprend un dispositif de tensionnement (21) pour la feuille (10), de préférence sous la forme d'un accumulateur de force à ressort (21 ").

7. Dispositif selon l'une des revendications 1 à 6, **caractérisé en ce que** la feuille (10) comprend un renfort textile ou est doublée d'un renfort textile.

8. Dispositif selon l'une des revendications 1 à 7, **caractérisé en ce que** la feuille (10) est pourvue d'une surface d'appui (10') avec de bonnes propriétés de coulissement.

9. Dispositif selon l'une des revendications 1 à 8, **caractérisé en ce que** la feuille (10) est réalisée au moins en deux couches, et comprend une feuille (10a) située tout en haut avec de bonnes propriétés de coulissement, et une feuille portante (10b) située au-dessous, qui comprend de préférence un renfort textile.

10. Dispositif selon la revendication 8, **caractérisé en ce que** la feuille (10a) située tout en haut et la feuille (10b) située au-dessous sont reliées en réalisant une cavité fermée et étanche (161) dans une région périphérique, c'est-à-dire qu'elles sont de préférence cousues, soudées, collées ou vulcanisées au niveau de leur zone de bordure périphérique (17, 17').

11. Dispositif selon la revendication 9 ou 10, **caractérisé en ce que**, entre la feuille (10a) située tout en haut avec de bonnes propriétés de coulissement et la feuille qui se trouve au-dessous (10b) en encaissant suffisamment les forces portantes, il est formé une cavité (161) qui peut être remplie avec un milieu, de préférence un milieu gazeux, liquide, pâteux et/ou en forme de gel, ou est susceptible d'être remplie via une ouverture de remplissage de préférence obturable.

12. Dispositif selon l'une des revendications 9 à 11, **caractérisé en ce que** la cavité (161) est remplie avec un milieu de telle façon ou bien est susceptible d'être remplie via une ouverture d'admission obturable, et en cas de besoin d'être vidée totalement ou partiellement de telle façon que grâce à la feuille (10b) légèrement en suspension et assurant la fonction portante, et grâce au degré de remplissage dans la cavité (161), la feuille coulissante (10, 10a) située tout en haut est susceptible d'être réglée quant à sa position, de préférence horizontalement, quasi horizontalement ou avec une faible flexion concave qui peut être préalablement choisie.

13. Dispositif selon l'une des revendications 1 à 12, **caractérisé en ce qu'**il est prévu une autre feuille (10c), laquelle est agencée au-dessous de la feuille (10b) qui remplit la fonction portante.

14. Dispositif selon la revendication 13, **caractérisé en ce que** la feuille inférieure (10c) est fermement reliée, cousue, soudée, collée, etc., avec la feuille (10b) et de préférence également avec la feuille (10a) dans une périphérie extérieure, de préférence au niveau de sa bordure (17, 17') en réalisant une cavité (163).

15. Dispositif selon la revendication 13 ou 14, **caractérisé en ce que** la cavité (163) est susceptible d'être remplie avec un fluide thermique et/ou **en ce qu'**elle est susceptible d'être chauffée par un fluide de chauffage en écoulement tout autour.

16. Dispositif selon la revendication 15, **caractérisé en ce que** la feuille en suspension vers le bas (10c) assure également une fonction portante, de préférence en utilisant un renfort textile.

17. Dispositif selon l'une des revendications 1 à 16, **caractérisé en ce que** la feuille (10) est tenue, de préférence avec les feuilles individuelles (10a, 10b, 10c) grâce à un dispositif porteur (1) doté de moyens pour se tenir debout ou de moyens pour poser les pieds.

18. Dispositif selon l'une des revendications 1 à 17, **caractérisé en ce qu'**il est prévu un dispositif de levage/abaissement pour la feuille (10).

19. Dispositif selon l'une des revendications 1 à 18, **caractérisé en ce que** la feuille (10) adopte, lors de l'appui d'une personne, une légère forme en coquille ou en poêle dite "wok".

20. Dispositif selon l'une des revendications 1 à 19, **caractérisé en ce qu'**il est prévu des inserts (43) dans le compartiment à feuille (1"), qui coopèrent avec la face inférieure des feuilles.

21. Dispositif selon la revendication 20, **caractérisé en ce que** les inserts (43) sont en matériau élastique et/ou déformable.

22. Dispositif selon la revendication 21, **caractérisé en ce qu'**au moins un insert (43) est en caoutchouc mousse ou en une mousse ou encore un matériau comparable, de sorte qu'une personne qui se trouve sur la feuille (10) repose depuis le côté supérieur sur l'insert (43) ainsi formé, de préférence avec une compression au moins légère de cet insert.

23. Dispositif selon la revendication 20, **caractérisé en ce que** l'insert (43) est en un granulé de préférence capable de flotter.

24. Dispositif selon l'une des revendications 1 à 20, **caractérisé en ce que** le dispositif porteur (1) comprend un corps de base (1') en cuvette, et **en ce que** l'espace entre la face inférieure de la feuille (10) et le volume intérieur (1") de la cuvette est susceptible de recevoir un écoulement de milieu liquide et/ou gazeux, de préférence sous la forme d'un système en recirculation en utilisant au moins un tube d'admission et au moins un tube d'évacuation (45, 46).

25. Dispositif selon l'une des revendications 1 à 24, **caractérisé en ce qu'**au moins deux moyens de maintien et/ou de saisie (47) peuvent être amenés, pivotés et/ou renversés depuis l'extérieur au moins jusque dans la région de bordure de la feuille (10), et de préférence susceptibles d'être fixés dans cette position.

26. Dispositif selon l'une des revendications 1 à 25, **caractérisé en ce qu'**un moyen de soutien latéral (49), séparé de la feuille (10) et de préférence rembourré et/ou une partie intérieure (50) de préférence élastique, souple et/ou rembourrée et/ou une partie repose-genoux (50) est prévu(e) sur la feuille (10).

27. Dispositif selon la revendication 16, **caractérisé en ce que** ledit au moins un moyen de soutien (49) et/ou une partie intérieure ou partie repose-genoux (50) sont susceptibles d'être pivotés vers l'extérieur depuis une position d'utilisation qui se trouve sur la feuille coulissante (10) jusque dans une position hors d'engagement.

28. Dispositif selon la revendication 26 ou 27, **caractérisé en ce que** la partie intérieure ou partie repose-genoux (50) est capable de pivoter de préférence autour d'un repose-pieds (48, 48').

29. Dispositif selon l'une des revendications 26 à 28, **caractérisé en ce que** ledit au moins un moyen de soutien (49) et/ou la partie intérieure ou partie repose-genoux (50) sont dotés de moyens de saisie (47').

30. Dispositif selon l'une des revendications 1 à 29, **caractérisé en ce qu'**il est prévu un système manuel et/ou motorisé qui met en déplacement au moins le dispositif de maintien et de renvoi de la feuille (10) et de préférence la totalité du dispositif porteur (1) respectivement du corps de base en cuvette (1').

31. Dispositif selon la revendication 30, **caractérisé en ce que** ledit système permet un mouvement de translation, un mouvement de basculement ou de pivotement et/ou au moins un mouvement de rotation au moins de la feuille coulissante (10), c'est-à-dire de préférence y compris le dispositif de renvoi (11) qui la porte.

32. Dispositif selon la revendication 31, **caractérisé en ce que** le système correspondant est prévu au-dessous du dispositif porteur (1) ou du corps de base en cuvette (1').

33. Dispositif selon l'une des revendications 14 à 16, **caractérisé en ce que** la cavité (163) est susceptible d'être enflée de telle manière que la feuille (10a) située tout en haut adopte une courbure convexe pour permettre l'évacuation latérale de l'eau de nettoyage.
